# EUROPÄISCHE PATENTANMELDUNG

(11) **EP 0 951 878 A1**
(43) Veröffentlichungstag der Anmeldung: **27.10.1999**
(21) Anmeldenummer: 99108099.5
(22) Anmeldetag: 23.04.1999
(51) Int. Cl.: A61F 2/16, A61N 5/10, A61F 2/14

(54) **Implantat in den Kapselsack in Verbindung mit einer Kataraktoperation**

(30) Priorität: 24.04.1998 DE 19818487
(71) Anmelder: Joussen, Antonia J.M. Dr., 53474 Bad Neuenahr (DE)
(72) Erfinder: Joussen, Antonia J.M. Dr., 53474 Bad Neuenahr (DE)
(74) Vertreter: Dr. Elisabeth Jung Dr. Jürgen Schirdewahn Dipl.-Ing. Claus Gernhardt

(57) **Zusammenfassung**

Die Erfindung bezieht sich auf ein Implantat (22) in den Kapselsack (14) in Verbindung mit einer Kataraktoperation. Nach der Erfindung ist das Implantat (22) mit ionisierende Strahlung aussendenden Nukliden beladen.

## Beschreibung

Die Erfindung bezieht sich auf ein Implantat in den Kapselsack in Verbindung mit einer Kataraktoperation. Bekannte derartige Implantate sind eine Intraokularlinse, gegebenenfalls in Verbindung mit einer Haptik, und/oder ein Kapselspannring.

Die Erfindung befaßt sich mit einer Proliferationshemmung von Linsenepithelzellen sowie anderen proliferierenden Zellen (Irispigmentepithelzellen, Makrophagen etc.) im Bereich der hinteren Linsenkapsel (respective des Kapselsackes) des menschlichen Auges.

Limitierend nach Kataraktextraction ist häufig die Bildung eines Nachstars. Der sogenannte regeneratorische Nachstar besteht aus einer makroskopisch groben Wucherung von bei der Operation im Kapselsack verbliebenen Linsenepithelzellen, die beim Einwachsen in die optische Achse zu einer Sehverschlechterung führen.

Bei der zur Zeit am häufigsten angewendeten Neodymium YAG-Laser Therapie wird durch eine Photodisruption die Hinterkapsel zerrissen. Diese Behandlungsmethode ermöglicht in den meisten Fällen eine effektive Wiederherstellung der Sehschärfe, beinhaltet jedoch auch Risiken wie die Entstehung einer Netzhautablösung oder von Regenbogenhautentzündungen. Die Zerstörung der Hinterkapsel würde zudem durch die Ruptur der intakten Hinterkapsel die Verwendung eines flüssigen Linsenersatzes als Implantat der Zukunft verbieten.

Im Laufe der letzten Jahre wurde daher immer wieder nach neuen Methoden gesucht, die, möglichst ohne andere okuläre Gewebe zu schädigen, die proliferierenden Linsenepithelzellen vollständig entfernen können oder sie selektiv derart schädigen, daß eine weitere Zellvermehrung verhindert wird. Während chirurgische Methoden der Kapselpolitur sich als nicht ausreichend erweisen, haben pharmakologische Verfahren oder enzymatische Verfahen den Nachteil der Diffusion toxischer Substanzen in die Vorderkammer, was zu irreversiblen Schäden des sehr empfindlichen Hornhautepithels führt (siehe: Greite 1989, Caldwell 1984, Nishi 1991, Nishi 1993, Knorr 1992, Knaus 1990, Xia 1994).

Aus der Kardiologie ist es schon bekannt, sogenannte -stents 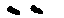 dazu verwendet, mit Hilfe eines im Gefäßlumen plazierten Drahtschlingennetzes dieses offenzuhalten. Seit mindestens zehn Jahren haben Untersuchungen ergeben, daß durch das Einsetzen radioaktiver -stents 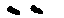 eine deutlich höhere Effektivität erzielt werden konnte. Diese radiaoktiven -stents 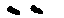 bestehen beispielsweise aus Edelstahl, welcher mit dem β-Strahler Phosphor 32 aktiviert wird. Die durch diese β-Strahlung beeinflußten Endothelzellen und die Gefäßverschlußeffekte, die durch Zuwuchern durch die Endothelzellen verhütet werden sollen, sind jedoch mit den Linsenepithelzellen und deren befürchteter Überwucherung des hinteren Bereichs der Linsenkapsel im Zusammenhang mit einer Kataraktoperation nicht vergleichbar.

Bei der Augenheilkunde hat man bisher nur externe radioaktive Strahlungsquellen entweder diagnostisch oder auch therapeutisch, z.B. zur Tumorbehandlung, eingesetzt. Die Erfahrung bestand dabei jedoch, daß die Auswirkung einer derartigen externen Bestrahlung sich in einer Trübung des Linsenkörpers eines noch gesunden Auges äußern kann. Auch bei der experimentellen Bestrahlung von Proben von Linsenepithelzellen in vitro, die zu einer Hemmung der Zellteilung führte, hat man bisher nur gesonderte ionisierende Strahlungsquellen konventioneller Art eingesetzt, um die Zellreaktion in Abhängigkeit von der Strahlungsdosis zu bestimmen.

Der Erfindung liegt die Aufgabe zugrunde, eine unerwünschte Proliferation von Linsenepithelzellen in vivo zu hemmen und dabei die genannten Schädigungen bekannter Methoden für diesen Zweck weitmöglichst zu vermeiden.

Diese Aufgabe wird durch ein Implantat gemäß Anspruch 1 gelöst.

Die Erfindung zieht erstmalig die Anwendung von Strahlenträgern im Kapselsack zur Proliferationshemmung von Linsenepithelzellen in Betracht. Parallelen zu den in der Kardiologie verwendeten radioaktiven stents bestehen nur bezüglich der verwendbaren Strahler. An intraokulare Implantate sind jedoch von Konfiguration und Material her andere Anforderungen gestellt.

Gemäß den Unteransprüchen zieht die Erfindung sowohl eine Beladung mit ionisierender Strahlung aussenden Nukliden bei schon bekannten Implantaten, wie an der Intraokularlinse, gegebenenfalls auch deren Haptik, oder an einem in Verbindung mit einer Intraokularlinse selbständig eingesetzten Kapselspannring in Betracht, wie auch an einem Ergänzungsimplantat gemaß Anspruch 10. Ein solches Ergänzungsimplantat kann seinerseits z.B. ring- oder plattenförmig geformt sein.

Die Art des Implantats, die Auswahl der Radionuklide sowie Art, Form und Anbringungsweise der Radionuklide im Implantat lassen sich weitgehend variieren und an die Bedürfnisse eines speziellen Patienten anpassen. Dabei kann man auch in Betracht ziehen, das mit einem bestimmten Radionuklid oder einer Kombination derselben beladene Implantat nicht permanent, sondern nur temporär im Kapselsack des Patienten zu belassen. Bei diesen Maßnahmen kommt es insbesondere auf das Alter des Patienten, dessen zelluläre Konstitution und eventuelle Vorerfahrungen von Zellwucherungen im Kapselsack an, um wirkungsvoll die Ausbildung eines Nachstars solange wie möglich zu verhindern.

Speziell kann je nach befürchtetem Nachstar eine unterschiedliche Strahlungsdauer, die zwischen Stunden und mehreren Jahren variieren kann, z.B. je nach dem Lebensalter des Patienten, vorgesehen werden.

Im Rahmen der Erfindung können unterschiedliche Trägerelemente (Kunststoffe, Acryl, Metalle wie Edelstahl oder Platin) als Bestandteil des Implantates in unterschiedlicher Konfiguration verwendet werden. Daneben können unterschiedliche radioaktive Elemente (β-Strahler, kurz oder langlebig:, z.B. Yttrium 90, oder Phosphor 34 oder Strahler mit einer Eindringtiefe von max. 2-3 mm Anwendung finden, damit die umliegenden Gewebe nicht geschädigt werden. Die hierbei verwendete Gesamtstrahlungsdosis im Bereich von 5-25 Gy hat sich als geeignet erwiesen, ohne daß die Erfindung auf diesen bevorzugten Dosisbereich beschränkt sein soll.

Die Implantate können nach Abklingen der Strahlung in situ verbleiben.

Die Erfindung wird im folgenden anhand schematischer Zeichnungen an mehreren Ausführungsbeispielen noch näher erläutert. Es zeigen:
Fig. 1 eine Ansicht eines bei einer Kataraktoperation eröffneten Kapselsackes, zunächst noch ohne Einsetzung eines Implantats;
Fig. 2 eine Ansicht gemäß Fig. 1 mit bereits eingesetztem Implantat in schematischer plattenförmiger Darstellung;
Fig. 3a eine Draufsicht auf ein Implantat in Gestalt einer Intraokularlinse mit Haptik;
Fig. 3b eine Seitenansicht des Implantats gemäß Fig. 3a;
Fig. 4 eine Draufsicht auf ein Implantat in Gestalt eines Kapselspannrings;
Fig. 5a eine Draufsicht auf ein Ergänzungsimplantat; und
Fig. 5b eine Seitenansicht des Ergänzungsimplantats nach Fig. 5a.

Die nachfolgend beschriebenen Implantate können alle ganz oder teilweise mit ionisierende Strahlung aussendenden Nukliden beladen sein, die ihrerseits wiederum in einer Beschichtung und/oder im eigentlichen Material des Implantates selbst eingebettet sein können.

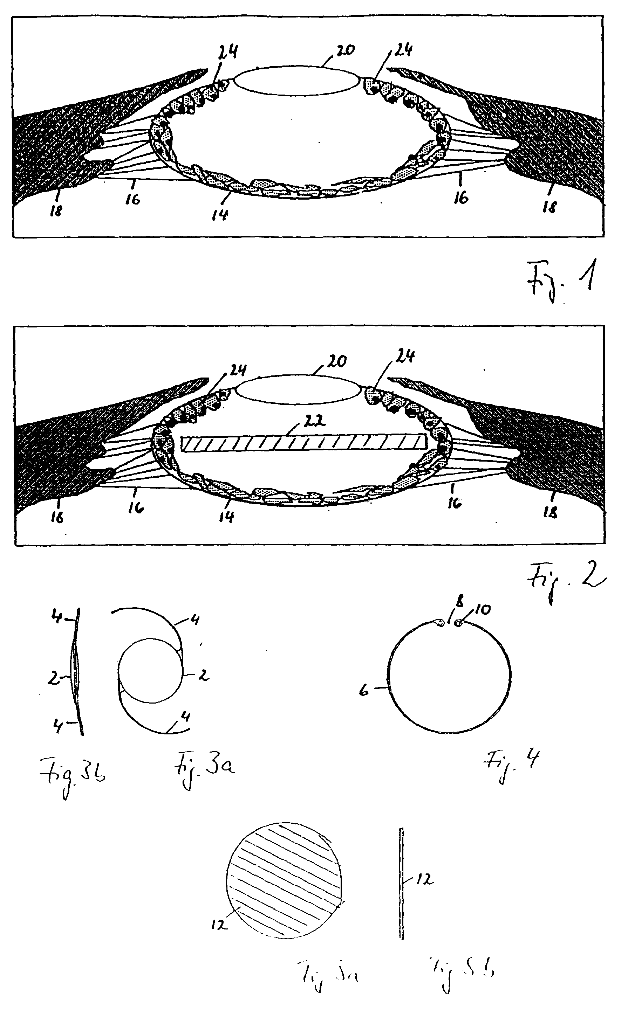

In diesem Zusammenhang zeigen die Fig. 3a und 3b eine Intraokularlinse 2 mit beidseitig diametral von der Intraokularlinse ausgehender Haptik 4. Für die Haptik ist hier eine beliebige Auswahl aus einer Vielzahl bekannter Ausführungsformen einer Haptik ohne Beschränkung der Allgemeinheit dargestellt. Zum Beispiel kann in nicht dargestellter Weise die Haptik auch flächenhaft oder ringförmig an den Umfang der Intraokularlinse 2 anschließen. Insbesondere für die Intraokularlinse 2 kommt die Anordnung eines nuklidbeladenen Bereiches nur in einer peripheren, vorzugsweise ringförmig geschlossenen, Zone der Intraokularlinse in unmittelbarer Nachbarschaft der verbliebenen Linsenepithelzellen nach Entfernen des natürlichen Linsenkernes und der natürlichen Linsenrinde aus dem Kapselsack in Frage. Sonst kommt man oft mit Anbringung der Radionuklide in Beimischung zum Material des Implantats und/oder einfach zusammenhängender flächenmäßiger Beschichtung aus.

Fig. 4 zeigt einen Kapselspannring 6, hier speziell in Ausbildung als gespaltener Ring mit Spalt 8 und diesen begrenzenden, in den spaltbildenden Enden des Kapselspannrings ausgebildeten Positionierlöchern 10.

Fig. 5 zeigt ein Ergänzungsimplantat 12, das hier ohne Beschränkung der Allgemeinheit als flache Kreisscheibe ausgebildet ist. In dieser Ausführungsform kann man das Ergänzungsimplantat 12 in den Kapselsack zwischen im Strahlengang hinter einem refraktiv wirksamen Implantat wie z.B. der Intraokularlinse 2 implantieren, und zwar sowohl für sich als auch in gegenständlicher Kombination mit dem refraktiven Implantat 12.

Die Implantatstechnik aller solcher Implantate in den Kapselsack 14 wird anhand der Fig. 1 und 2 grob schematisch veranschaulicht.

Der Kapselsack 14 wird in einem Auge durch Zonulafasern am Ziliarkörper gehalten.

Bei einer Kataraktoperation wird der Kapselsack 14 oben unter Bildung einer runden Eingriffsöffnung 20 (Kapsulorhexis) eröffnet. Durch diese Eingriffsöffnung kann das generalisiert dargestellte Implantat 22 eingesetzt werden, das für alle Arten der Implantate gemäß den Fig. 3a bis 5b und auch eine Kombination derselben stehen kann.

Für die Funktion der Erfindung wesentlich ist die Tatsache, daß nach Einsetzen des Implantats 22 in den Kapselsack 14 dessen Umfang an verbliebene Linsenepithelzellen 24 direkt oder mit Zwischenabstand angrenzt. Durch die Beladung des Implantats mit den Radionukliden soll eine Veränderung des Kapselsackes 14 in optischer Klarheit und/oder Konfiguration durch proliferierende Linsenepithelzellen verhindert werden.

### Literatur:

Amols HI, Reinstein LE, Weinberger J (1996) Dosimetry of a radioaktive coronary bolloon dilatation catheter for treatment of neointimal hyperplasia. Med- Phys 23: 1783-1788
Caldwell D (1984) Freezing of the posterior capsule in extracapsular extraction. Highlights of Opthalmol letter 12:7
Fine IH (1991) The chip and flip phakoemulsification technique. J Cat Refr Surg 17: 366-371
Greite JK, Kaden P, Kreiner CF (1989) -Osmolavage 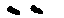 zur Nachstarverhütung. In: Freyler H, Skorpik C, Grasl M (eds.): 3. Kongress der Dt. Gesellschaft für Intraokularlinsen-Implantation: Wien, New York Springer
Hansen TJ, Tyndall R, Soll DB (1987) Methotrexade-antikollagen conjugate inhibits in vitro lens cell outgrowth. Invest Ophthalmol 86: 167-171
Hehrlein C, Stinz M, Kinscherf R, Schlosser K, Huttel E, Friedrich L, Fehsenfeld P, Kübler W (1996) Pure beta-particle-emitting stends inhibit neointima formation in rabbits. Circulation 15: 641-645
Joussen AM, Kruse FE, Rohrschneider K, Völcker HE (1995) Devitalisierung von Linsenepithelzellen durch Farbstoff-verstärkte Therapie. Ophthalmologe 92: 581-590
Joussen AM, Kruse FE, Kaus M, Völcker HE (1997) Endogene Porphyrine zur photodynamischen Therapie des Nachstars in vitro Ophthalmologe 94: 428-435
Knorr M, Wunderlich K, Steul K-P, Thiel H-J, Dartsch PC (1992) Wirkung von Heparin auf die Proliferation kultivierter boviner Linsenepithelzellen. Ophthalmologe 89: 319-324
Knaus HG, Scheffauer F, Romanin C, Schindler HG, Glossmann H (1990) Heparin binds with high affinity to voltage dependent L-type Ca2+ channels. J Biol Chem 265: 11156-11166
Maguen E, Martinez M, Grundfest W (1989) Excimer laser ablation of the human lens at 308 nm with a fiber delivery system. J Cat Refr Surg 15: 409-415
Marshall J, Sliney DH (1986) Endoexcimer laser intraocular ablative photodecomposition. Letter. Am J Ophthalmol 101: 130-131
Nishi O, Nishi K, Hikida M (1991) Removal of lens epithelial calls by dispersion with enzymatic treatment followed by aspiration. Ophthalmic Surg 22: 444-450
Nishi O, Nishi K, Hikida M (1993) Removal of lens epithlial cells following loosening of the junctional complex. J Cat Refract Surg 19: 56-61
Power WJ, Neylan D, Collum LM (1994) Adherence of human lens epithleila cells to conventional polymethyl methacrylate, heparin - surface modified, and poly-Hema lenses. J Cat Refr Surg 20: 440-445
Shen J, Smon G, Parel J-M, Ren Q (1993) Investigation of laser-phaco devices for cataract surgery. Invest Ophthalmol Vis Sci 34: 1452
Tetz MR, O'Morchoe DJC, Todd DG, Wilbrandt TH, Solomon KD, Hansen SO, Apple DJ (1988) Posterior capsule opacification and intraocular lens decentration. Part II: Experimental findings on a prototype circular intraocular lens design. J Cat Refr Surg 14: 614-623
Xia XP, Lu DY, Wang LT (1994) A clinical study of inhibition of secondary cataract with heparin. Chin J Ophthalmol 30: 405-407

## Patentansprüche

1. Implantat (22) in den Kapselsack (14) in Verbindung mit einer Kataraktoperation, **gekennzeichnet** durch Beladung mit ionisierende Strahlung aussendenden Nukliden.

2. Implantat nach Anspruch 1, dadurch gekennzeichnet, daß die Nuklide mindestens teilweise β-Strahler sind.

3. Implantat nach Anspruch 2, dadurch gekennzeichnet, daß die Nuklide Yttrium 90, Phosphor 34 und/oder Strontium 90 sind.

4. Implantat nach einem der Ansprüche 1 bis 3, dadurch gekennzeichnet, daß mindestens ein nuklidbeladener Bereich an einer Intraokularlinse (2) ausgebildet ist.

5. Implantat nach Anspruch 4, dadurch gekennzeichnet, daß dieser nuklidbeladene Bereich mindestens von einem Teilvolumen der Intraokularlinse (2) unter Beimischung der Nuklide zu deren Material gebildet ist.

6. Implantat nach Anspruch 5, dadurch gekennzeichnet, daß dieser nuklidbeladene Bereich ein peripherer, vorzugsweise ringförmig geschlossener, Bereich der Intraokularlinse (2) ist.

7. Implantat nach einem der Ansprüche 4 bis 6, dadurch gekennzeichnet, daß dieser nuklidbeladene Bereich sich über die rückseitige Oberfläche der Intraokularlinse (2) als Beschichtung erstreckt.

8. Implantat nach einem der Ansprüche 1 bis 7, dadurch gekennzeichnet, daß ein nuklidbeladener Bereich an einer außerhalb des refraktiven Bereichs angebrachten Linsenhaptik (4) ausgebildet ist.

9. Implantat nach einem der Ansprüche 1 bis 8, dadurch gekennzeichnet, daß ein nuklidbeladener Bereich an einem Kapselspannring (6) ausgebildet ist.

10. Implantat nach einem der Ansprüche 1 bis 9, dadurch gekennzeichnet, daß ein nuklidbeladener Bereich an einem im Kapselsack (14) anordbaren, aber von der Intraokularlinse (2), deren Haptik (4) und einem Kapselspannring (6) unabhängigen Ergänzungsimplantat (12) ausgebildet ist, das in Verbindung mit oder unabhängig von Intraokularlinse (2), deren Haptik (4) und einem Kapselspannring (6) in den Kapselsack (14) implantierbar ist.

11. Implantat nach einem der Ansprüche 8 bis 10, dadurch gekennzeichnet, daß der nuklidbeladene Bereich als Beschichtung und/oder Beimengung des Materials ausgebildet ist.
